# EUROPEAN PATENT SPECIFICATION

(11) **EP 1 744 733 B1**
(45) Date of publication and mention of the grant of the patent: **19.09.2018**
(21) Application number: 05745455.5
(22) Date of filing: 05.05.2005
(51) Int. Cl.: A61K 9/48

(54) **SOFTGEL ENCAPSULATED PHARMACEUTICAL COMPOSITIONS COMPRISING CONCENTRATED ACTIVE INGREDIENTS**
IN SOFTGEL VERKAPSELTE PHARMAZEUTISCHE ZUSAMMENSETZUNGEN MIT KONZENTRIERTEN WIRKSTOFFEN
COMPOSITIONS PHARMACEUTIQUES ENCAPSULÉES DE GEL SOUPLE CONTENANT DES INGRÉDIENTS ACTIFS CONCENTRÉS

(30) Priority: 06.05.2004 US 840143
(43) Date of publication of application: 24.01.2007
(73) Proprietor: The Procter & Gamble Company, Cincinnati, OH 45202 (US)
(72) Inventor: KHANOLKAR, Jayant, Ekanth, Surbiton Surrey KT5 8RD (GB); DE LA HARPE, Shane, Michael, Liskeard Cornwall PL14 4BP (GB)
(74) Representative: Töpert, Verena Clarita
(86) International application number: PCT/US2005/015746
(87) International publication number: WO 2005/107709

(56) References cited:
- US-A- 5 002 777
- US-A- 5 484 606
- US-B1- 6 387 400

## Description

### FIELD OF THE INVENTION

The present invention is directed to pharmaceutical compositions comprising concentrated active ingredients. In particular, the present invention is directed to pharmaceutical compositions comprising highly concentrated pharmaceutical actives, wherein the pharmaceutical compositions are suitable for incorporation into soft gelatin capsules having improved stability.

### BACKGROUND OF THE INVENTION

Pharmaceutical compositions are available in many forms including liquid and solid pharmaceutical formulations. These pharmaceutical compositions are known to be effective in treating ailments such as symptoms of the common cold and/or influenza, and can be administered in various product forms such as liquid elixirs, encapsulated soft gelatin capsules, encapsulated hard shell capsules, tablets, lozenges, and so forth.

Technology related to the administration of liquid pharmaceutical compositions has advanced such that pharmaceutical compositions comprising highly concentrated active ingredients have been successfully formulated to treat cold and/or influenza-like symptoms. See, for example, U.S. Patents 5,484,606; 5,141,961; 5,641,512; and 6,251,426; WO 95/19759; WO 03/013481; and WO 95/04527. The liquid pharmaceutical compositions comprising highly concentrated active typically contain a combination of polyethylene glycol and polyvinylpyrrolidone solvents to solubilize the concentrated active.

The use of soft gelatin capsules (also known as "softgels") to encapsulate liquid pharmaceutical compositions comprising highly concentrated active is also known. The liquid pharmaceutical compositions contained within the soft gelatin capsules are typically compositions comprising solubilized or suspended concentrated active ingredients. The soft gelatin capsules are suitable for use as vitamin supplements, for the prevention and treatment of symptoms of the common cold and/or influenza, and for other pharmaceutical treatments such as antacid capsules, benzodiazepine capsules, antiflatulent capsules, and so forth.

One attempt to manufacture a soft gelatin capsule containing a liquid pharmaceutical composition that comprises a suspension of concentrated active is disclosed in U.S. Patent 5,002,777. This patent relates to the encapsulation of a concentrated liquid suspension of calcium carbonate particles of specific size and shape, wherein the capsules are suitable for use as antacid capsules.

Another attempt to manufacture a soft gelatin capsule containing a liquid pharmaceutical composition that comprises a suspension of concentrated active is the process disclosed in U.S. Patent 6,387,400. This patent discloses a process for formulating suspensions of increased concentration of pharmaceutical active ingredients by mixing active-containing suspensions with a hydroxide ion source.

Yet other attempts to manufacture soft gelatin capsules containing liquid pharmaceutical compositions comprising suspensions of active ingredients are described in U.S. Patents; 5,908,636; 5,916,590; 5,919,481; and 6,024,980. These patents disclose soft gelatin capsules filled with a semi-solid containing a therapeutically effective amount of a pharmaceutical active.

Still yet another attempt to manufacture soft gelatin capsules containing liquid pharmaceutical compositions that comprise concentrated active ingredients is disclosed in U.S. Patent 5,360,615, which describes a solvent system comprising concentrated active and an ionizing agent wherein the solvent system is encapsulated within a softgel.

There also exists prior disclosure outlining the stability effect of soft gelatin capsules comprising a hydrophilic suspension fill that contains acetaminophen. See, for example, the publication by Karunakar S., John T., Chidambaram N., and Dale K.; Banner Pharmacaps, Inc.; entitled "Effect of acetaminophen concentration, as a hydrophilic suspension fill, on the stability of soft gelatin capsules". This publication demonstrated that soft gelatin capsules comprising a high dose of acetaminophen can exhibit deteriorating physical changes such as seam widening, softening, sticking, and increase in the gelatin shell weight.

Despite the state of the art in describing soft gelatin capsules filled with liquid pharmaceutical compositions comprising concentrated active ingredients, there is no disclosure of such soft gelatin capsules having improved stability. It has been found that specific stabilizing agents can be incorporated into liquid compositions to provide for improved stability of the compositions and soft gelatin capsules containing the compositions. The stabilizing agents have been found to prevent or minimize negative capsule attributes such as hydrolyzation, premature dissolution, seam widening, softening, sticking, increase shell weight, or tanning of soft gelatin capsules. In addition, the stabilizing agents have been found to provide for minimal or no oxidation or degradation of the active ingredient fill or gelatin shell.

### SUMMARY OF THE INVENTION

The present invention is directed to a pharmaceutical composition that comprises (a) from 55% to 90% by weight of a suspended pharmaceutical active; (b) from 0.001% to 1.00% by weight of a suspended stabilizing agent; selected from disodium salts of ethylene diamine tetraacetic acid, calcium salts of ethylene diamine tetraacetic acid, tetrasodium ethylene diamine tetraacetic acid and mixtures thereof; and (c) from 9% to 39% by weight of a solvent selected from polyethylene glycols; wherein the composition is encapsulated within a soft gelatin capsule.

It has been found that the stability of soft gelatin capsules can be improved by the incorporation of select stabilizing agents into liquid pharmaceutical compositions encapsulated within the soft gelatin capsules. It is known that liquid pharmaceutical compositions comprising concentrated active ingredients can be encapsulated within soft gelatin capsules, however, such soft gelatin capsules have a tendency to exhibit instability properties such as hydrolyzation, premature dissolution, seam widening, softening, sticking, increase shell weight, and/or tanning (hardening). It has been found that the stabilizing agents of the present invention provides for improved stability of soft gelatin capsules, especially soft gelatin capsules that contain liquid pharmaceutical compositions that comprise suspended, highly concentrated active ingredients.

### DETAILED DESCRIPTION OF THE INVENTION

The pharmaceutical compositions of the present invention are liquid pharmaceutical compositions that comprise a pharmaceutical active and stabilizing agent suspended within a solvent, wherein the compositions are encapsulated within a soft gelatin capsule. These soft gelatin capsules are especially effective in the prevention and/or treatment of cold and influenza-like symptoms.

The terms "suspended and suspension" are used interchangeably herein to refer to solutions comprising at least solvent, pharmaceutical active, and stabilizing agent, wherein the active and/or stabilizing agent are dispersed, not dissolved or solubilized, within the solvent.

The term "treatment of cold and influenza-like symptoms" as used herein refers to treating the onset, duration, and/or after-effect of cold and influenza-like symptoms. In other words, "treatment of cold and influenza-like symptoms" includes preventing the onset of cold and influenza-like symptoms, and alleviating cold and influenza-like symptoms.

As used herein "cold and influenza-like symptoms" refer to symptoms typically associated with respiratory tract viral infections. These symptoms include, but are not limited to, nasal congestion, chest congestion, sneezing, rhinorrhea, fatigue or malaise, coughing, fever, chills, body ache, sore throat, headache, and other known cold and influenza-like symptoms.

Cold and influenza-like symptoms typically result from respiratory tract viral infections caused by respiratory viruses. Therefore, the term "respiratory viruses" as used herein refers to those viruses that are causal agents of cold and influenza-like symptoms. These viruses include Rhinovirus, Myxovirus (Influenza virus), Paramyxovirus (Parainfluenza virus), Respiratory Syncytial virus, Adenovirus, and Coronavirus.

The pharmaceutical compositions of the present invention can comprise, consist of, or consist essentially of the elements and limitations of the invention described herein, as well as any of the additional or optional ingredients, components, or limitations described herein.

All percentages, parts and ratios are by weight of the pharmaceutical compositions, unless otherwise specified. All such weights as they pertain to listed ingredients are based on the specific ingredient level and, therefore, do not include carriers or by-products that may be included in commercially available materials, unless otherwise specified.

Citation of any document is not an admission regarding any determination as to its availability as prior art to the present invention.

### Pharmaceutical Active

The pharmaceutical compositions of the present invention comprise a pharmaceutical active. The pharmaceutical active can be included in the compositions as an individual active ingredient or as a combination of active ingredients. The pharmaceutical active is preferably a combination of active ingredients that are especially effective in the prevention and treatment of cold and influenza-like symptoms.

The pharmaceutical active is included in the pharmaceutical compositions of the present invention as a highly concentrated pharmaceutical active. In this context "highly concentrated" refers to an amount of pharmaceutical active that is included in the composition at increased concentration ranges, wherein the pharmaceutical compositions comprise highly concentrated pharmaceutical active at total active concentration ranges of from 55% to 90%, preferably from about 58% to about 80%, more preferably from about 60% to about 75%, by weight of the pharmaceutical composition.

Nonlimiting examples of active ingredients suitable for use as a highly concentrated pharmaceutical active herein include those active ingredients that are pharmacologically classified as antitussives, antihistamines, non-sedating antihistamines, decongestants, expectorants, mucolytics, analgesics, antipyretics, anti-inflammatory agents, local anesthetics, and mixtures thereof. These active ingredients are more fully described in J. G. Hardman, The Pharmacologic Basis of Therapeutics, Ninth Edition, McGraw-Hill, New York, 1995.

Specific nonlimiting examples of antitussives suitable for use as a highly concentrated pharmaceutical active herein include those antitussive compounds which are especially effective in treating symptoms of the common cold such as fits of coughing. Suitable specific antitussives include codeine, dextromethorphan, dextrorphan, hydrocodone, noscapine, oxycodone, pentoxyverine, and mixtures thereof. Dextromethorphan is the most preferred antitussive. As used herein, "dextromethorphan" means racemethorphan, (±)-3-Methoxy-17-methylmorphinan, dl-cis-1,3,4,9,10,10a-hexahydro-6-methoxy-11-methyl-2H-10,4a-iminoethanophenanthrene, and pharmaceutical salts thereof including dextromethorphan hydrobromide. Dextromethorphan and its pharmaceutically-acceptable salts are more fully described in U.S. Patent 5,196,436, issued to Smith on March 23, 1993.

Specific nonlimiting examples of antihistamines suitable for use as a highly concentrated pharmaceutical active herein include acrivastine, azatadine, brompheniramine, brompheniramine maleate, chlorpheniramine, chlorpheniramine maleate, clemastine, cyproheptadine, dexbrompheniramine, dimenhydrinate, diphenhydramine, diphenhydramine hydrochloride, hydroxyzine, meclizine, pheninamine, phenyltoloxamine, promethazine, pyrilamine, pyrilamine maleate, tripelennamine, triprolidine, doxylamine, doxylamine succinate, and mixtures thereof.

Specific nonlimiting examples of non-sedating antihistamines suitable for use as a highly concentrated pharmaceutical active herein include astemizole, cetirizine, ebastine, fexofenadine, loratidine, terfenadine, and mixtures thereof.

Specific nonlimiting examples of decongestants suitable for use as a highly concentrated pharmaceutical active herein include phenylpropanolamine, pseudoephedrine, pseudoephedrine hydrochloride, pseudoephedrine sulfate, ephedrine, phenylephrine, phenylephrine hydrochloride, oxymetazoline, and mixtures thereof.

Specific nonlimiting examples of expectorants suitable for use as a highly concentrated pharmaceutical active herein include ammonium chloride, guafenesin, ipecac fluid extract, potassium iodide, and mixtures thereof.

Specific nonlimiting examples of mucolytics suitable for use as a highly concentrated pharmaceutical active herein include acetylcysteine, ambroxol, bromhexine, and mixtures thereof.

Specific nonlimiting examples of analgesics, antipyretics, and anti-inflammatory agents suitable for use as a highly concentrated pharmaceutical active herein include acetaminophen, aspirin, sodium salicylate, salicylamide, diclofenac, diflunisal, etodolac, fenoprofen, flurbiprofen, ibuprofen, ketoprofen, ketorolac, nabumetone, naproxen, piroxicam, caffeine, ketorolac, indomethacin, meclofenamic acid, COX-2 inhibitors such as valdecoxib, celecoxib and rofecoxib, and mixtures thereof.

### Stabilizing Agent

The pharmaceutical compositions of the present invention comprise a stabilizing agent which provides for improved stability of the compositions and soft gelatin capsules containing the compositions. Soft gelatin capsules containing a pharmaceutical composition comprising the stabilizing agent defined herein exhibit minimal or no hydrolyzation, premature dissolution, seam widening, softening, sticking, increase shell weight, or tanning of the gelatin capsule. The stabilizing agent also provides for minimal or no oxidation or degradation of the active ingredient fill or gelatin shell.

The pharmaceutical compositions can comprise one or more stabilizing agents, provided that the total concentration of stabilizing agent ranges from 0.001% to 1.00%, preferably from about 0.01% to about 1.00%, more preferably from about 0.01% to about 0.1%, by weight of the composition.

Suitable stabilizing agents include phytic acid, disodium salts of ethylene diamine tetraacetic acid (EDTA), calcium salts of ethylene diamine tetraacetic acid, tetrasodium ethylene diamine tetraacetic acid, sodium hexametaphosphate (SHMP), di(hydroxyethyl)glycine, 8-hydroxyquinoline, and mixtures thereof. Disodium salts of ethylene diamine tetraacetic acid are preferred.

As stated herein, the stabilizing agent can provide for minimal or no oxidation of the active ingredient fill or gelatin shell.

### Solvent

The pharmaceutical compositions of the present invention are preferably liquid pharmaceutical compositions that comprise the pharmaceutical active and stabilizing agent described hereinabove suspended in a solvent. One or more solvent materials are suitable for use herein, provided that the solvent is capable of providing for a suspension of compositional ingredients and that the solvent is suitable for encapsulation within soft gelatin capsules.

The liquid pharmaceutical compositions comprise one or more solvent at a total solvent concentration of from 9% to 39%, preferably from about 20% to about 39%, more preferably from about 30% to about 39%, by weight of the liquid pharmaceutical composition.

Nonlimiting examples of solvents suitable for use herein include polyethylene glycols, polyvinylpyrrolidones, propylene glycols, propylene glycol laureates, glyceryl monolinoleates, glyceryl monooleates, diethylene glycol monoethyl ethers, C₈-C₁₀ triglycerides, fractionated coconut oils, and mixtures thereof. These solvents are more fully described in the CTFA Cosmetic Ingredient Handbook, First Edition (1988) and The Merck Index, Eleventh Edition (1989). Polyethylene glycols are the preferred solvent materials.

Specific nonlimiting examples of preferred polyethylene glycols include those polyethylene glycols which corresponds to the general formula:

H(OCH₂CH₂)ₙOH

wherein n has an average value of from 4 to 35, preferably from 5 to 30, more preferably from 5 to 20. These materials are polymers of ethylene oxide, and are also known as polyethylene oxides, polyoxyethylenes, and PEGs. The polyethylene glycols are typically designated by both their average molecular weight range and their average "n" value as specified in the above general formula. For example, polyethylene glycol 400, which is also known by the CTFA designation of PEG-8, has a number average molecular weight range (Mₙ) of from about 380 to about 420 and an average n value of between 8.2 and 9.1. The number average molecular weight (Mₙ) of the polyethylene glycols can be determined by known titration procedures used to determine the number of molecules having hydroxy-end groups wherein the Mₙ is calculated based on the weight of a given polyethylene glycol divided by the number of hydroxy-end group-containing molecules within the polyethylene glycol polymer.

Specific examples of polyethylene glycols suitable for use herein include those polyethylene glycols designated as PEG-5, PEG-6, PEG-8, PEG-12, PEG-14, PEG-18, PEG-20, PEG-32, and mixtures thereof. Preferred are those polyethylene glycols which have a number average molecular weight of from about 190 to about 1500, preferably from about 300 to about 1200, more preferably from about 400 to about 1000; and an average n value of from 5 to 35, preferably from 5 to 30, more preferably from 5 to 20. Specific examples of the most preferred polyethylene glycols include, but are not limited to, PEG-8 wherein n has an average value of about 8 (PEG-8 is also known as Carbowax® 400, which is available from Dow Chemicals); PEG-12 wherein n has an average value of about 12 (PEG-12 is also known as Carbowax® 600, which is available from Dow Chemicals); and PEG-20 wherein and n has an average value of about 20 (PEG-20 is also known as Carbowax® 900, which is available from Dow Chemicals).

### Water

The pharmaceutical compositions of the present invention can comprise water at concentrations ranging from about 0.1% to about 5%, preferably from about 1% to about 2.5%, by weight of the composition. It is known that water can interact with soft gelatin capsule materials, therefore, the pharmaceutical compositions of the present invention should comprise water at the concentrations defined hereinabove.

### Optional Components

The pharmaceutical compositions of the present invention may further comprise one or more optional components known or otherwise effective for use in pharmaceutical compositions, provided that the optional components are physically and chemically compatible with the compositional components described hereinabove as well as gelatin capsules, or do not otherwise unduly impair product stability, aesthetics, or performance. Optional components suitable for use herein include materials such as flavorants including anise, oil of peppermint, oil of clove, eucalyptus, lemon, lime, honey lemon, red fruit, mint, grapefruit, orange, and cherry cola; sensory agents including coolants, salivating agents, and warming agents; and coloring agents including dyes, lake colors, and pigments such as titanium dioxide and titanium dioxide coated mica. The optional components can be included in the pharmaceutical compositions at concentrations ranging from about 0.001% to about 20%, preferably from about 0.01% to about 10%, by weight of the composition.

### Method of Manufacture

The pharmaceutical compositions of the present invention may be prepared by any known or otherwise effective technique suitable for providing a pharmaceutical composition that provides a therapeutic benefit, especially a therapeutic benefit in the prevention and treatment of symptoms associated with the common cold and influenza. The pharmaceutical compositions are preferably formulated to comprise a pharmaceutical active and stabilizing agent suspended in a solvent, all described herein, wherein these compositions are then encapsulated within soft gelatin capsules to prevent and treat respiratory tract viral infections such as those associated with the common cold and influenza.

The pharmaceutical compositions of the present invention can be encapsulated within any known or otherwise effective soft gelatin capsule such as those soft gelatin capsules described in WO 95/19759, published July 27, 1995, Dadi J. Dhabhar.

A typical preparation of the pharmaceutical compositions of the present invention involves heating a solution of solvents such as PEG 400 and propylene glycol to 40°C, and then forming a suspension by adding pharmaceutical active ingredients such as dextromethorphan hydrobromide, acetaminophen and pseudoephedrine hydrochloride and a premix of a stabilizing agent such as disodium EDTA dissolved in water, thereafter removing the suspension from heat. The resultant suspension is then encapsulated within a soft gelatin capsule.

### Method of Use

The pharmaceutical compositions of the present invention are encapsulated within soft gelatin capsules for the administration of highly concentrated pharmaceutical active ingredients. The amount of pharmaceutical active ingredients that are administered will depend upon factors such as the type of pharmaceutical active and the desired treatment response, however, typically from about 400mgs to about 800mgs of highly concentrated pharmaceutical active is administered per soft gelatin capsule. It has been found that the pharmaceutical compositions of the present invention can be administered using soft gelatin capsules such that increased concentration of pharmaceutical active can be administered using one dosage regimen as compared to typical dosage regimens of two or more capsules.

A preferred method of using the pharmaceutical compositions of the present invention involves the administration of soft gelatin capsules to treat and prevent symptoms associated with the common cold and influenza. Typically, one soft gelatin capsule comprising from about 500mgs to about 800mgs of pharmaceutical active is administered one to three times per day to effectively prevent and treat cold and influenza-like symptoms.

It is contemplated that soft gelatin capsules comprising the pharmaceutical compositions of the present invention can be administered as daytime dosage regimens and/or as nighttime dosage regimens. In other words, the pharmaceutical compositions of the present invention can comprise active ingredients that are suitable for daytime administration, and/or active ingredients that are suitable for nighttime administration. As such, a dosage regimen can include the administration of one or more soft gelatin capsules comprising a pharmaceutical composition that comprises one or more actives that are typically suitable for treating symptoms during the day, and the administration of one or more soft gelatin capsules comprising a pharmaceutical composition that comprises one or more actives that are typically suitable for treating nighttime symptoms.

### EXAMPLES

The following examples further describe and demonstrate embodiments within the scope of the present invention. The examples are given solely for the purpose of illustration and are not to be construed as limitations of the present invention. All exemplified concentrations are weight-weight percents, unless otherwise specified.

The pharmaceutical compositions exemplified below are made by suspending one or more pharmaceutical actives and a stabilizing agent in a solvent such as PEG 400. These compositions are encapsulated within soft gelatin capsules, and are suitable for use in the prevention and treatment of cold and influenza-like symptoms.

**Pharmaceutical Compositions**

| **Component** | **Sample 1 (Wt. %)** | **Sample 2 (Wt. %)** | **Sample 3 (Wt. %)** | **Sample 4 (Wt. %)** | **Sample 5 (Wt. %)** |
|---|---|---|---|---|---|
| Dextromethorphan HBr¹ | 2.500 | 2.500 | 2.73 | 2.50 | 2.375 |
| Acetaminophen² | 54.167 | 54.167 | 59.09 | 54.167 | 51.46 |
| Pseudoephedrine HCl³ | 5.000 | 5.000 | 5.45 | 5.00 | 4.75 |
| Doxylamine Succinate⁴ | --- | 1.041 | 1.14 | --- | --- |
| PEG 400⁵ | 36.323 | 35.282 | 30.24 | 34.283 | 38.405 |
| Disodium EDTA⁶ | 0.010 | 0.010 | 0.02 | 0.05 | 0.01 |
| Deionised Water | 2.000 | 2.000 | 1.33 | 4.00 | 3.00 |

| | | | | | |
|---|---|---|---|---|---|
| Wt. % - weight percent 1 - dextromethorphan hydrobromide available from Reddie Cheminor located in India 2 - acetaminophen available from Rhodia located in France 3 - pseudoephedrine hydrochloride available from BASF located in the USA 4 - doxylamine succinate available from Iropharm (Honeywell) located in Ireland 5 - polyethylene glycol 400 available from Dow Chemicals located in the USA 6 - disodium EDTA available from E Merck AG located in Germany | | | | | |

## Claims

1. A pharmaceutical composition comprising:
(a) from 55% to 90% by weight of a suspended pharmaceutical active;
(b) from 0.001% to 1.00% by weight of a suspended stabilizing agent selected from disodium salts of ethylene diamine tetraacetic acid, calcium salts of ethylene diamine tetraacetic acid, tetrasodium ethylene diamine tetraacetic acid, and mixtures thereof; and
(c) from 9% to 39% by weight of a solvent selected from polyethylene glycols;
wherein the composition is encapsulated within a soft gelatin capsule.

2. The composition of Claim 1 wherein the composition comprises from 58% to 80% by weight of the suspended pharmaceutical active selected from antitussives, antihistamines, non-sedating antihistamines, decongestants, expectorants, mucolytics, analgesics, antipyretics, anti-inflammatory agents, local anesthetics, and mixtures thereof.

3. The composition of Claim 1 or Claim 2 wherein the composition comprises from 0.01% to 1.00% by weight of the suspended stabilizing agent.

4. The composition of any of the preceding claims wherein the composition comprises from 20% to 39% by weight of polyethylene glycols.

5. The composition of any of the preceding claims wherein the composition further comprises from 0.1% to 5% by weight of water.

6. A method of improving the stability of a soft gelatin capsule wherein the method comprises the steps of:
(a) formulating a pharmaceutical composition comprising:
i) from 55% to 90% by weight of a suspended pharmaceutical active;
ii) from 0.001% to 1.00% by weight of a suspended stabilizing agent selected from disodium salts of ethylene diamine tetraacetic acid, calcium salts of ethylene diamine tetraacetic acid, tetrasodium ethylene diamine tetraacetic acid, and mixtures thereof; and
iii) from 9% to 39% by weight of solvent selected from polyethylene glycols; and
(b) encapsulating the composition of (a) within the soft gelatin capsule.

7. The method of Claim 6 wherein the suspended pharmaceutical active is selected from antitussives, antihistamines, non-sedating antihistamines, decongestants, expectorants, mucolytics, analgesics, antipyretics, anti-inflammatory agents, local anesthetics, and mixtures thereof.

8. The method of Claims 6 or 7 wherein the pharmaceutical composition further comprises from 0.1% to 5% by weight of water.

## Patentansprüche

1. Arzneimittelzusammensetzung, umfassend:
(a) von 55 Gew.-% bis 90 Gew.-% einen suspendierten Arzneimittelwirkstoff;
(b) von 0,001 Gew.-% bis 1,00 Gew.-% ein suspendiertes Stabilisierungsmittel, ausgewählt aus Dinatriumsalzen von Ethylendiamintetraessigsäure, Calciumsalzen von Ethylendiamintetraessigsäure, Tetranatriumethylendiamintetraessigsäure, und Mischungen davon; und
(c) von 9 Gew.-% bis 39 Gew.-% ein Lösungsmittel, ausgewählt aus Polyethylenglykolen;
wobei die Zusammensetzung in einer Weichgelatinekapsel eingekapselt ist.

2. Zusammensetzung nach Anspruch 1, wobei die Zusammensetzung 58 Gew.-% bis 80 Gew.-% suspendierten Arzneimittelwirkstoff, ausgewählt aus Antitussiva, Antihistaminen, nicht-sedierenden Antihistaminen, Dekongestiva, Expektoranzien, Mukolytika, Analgetika, Antipyretika, entzündungshemmenden Mitteln, Lokalanästhetika, und Mischungen davon, umfasst.

3. Zusammensetzung nach Anspruch 1 oder Anspruch 2, wobei die Zusammensetzung von 0,01 Gew.-% bis 1,00 Gew.-% das suspendierte Stabilisierungsmittel umfasst.

4. Zusammensetzung nach einem der vorstehenden Ansprüche, wobei die Zusammensetzung von 20 Gew.-% bis 39 Gew.-% Polyethylenglykole umfasst.

5. Zusammensetzung nach einem der vorstehenden Ansprüche, wobei die Zusammensetzung ferner von 0,1 Gew.-% bis 5 Gew.-% Wasser umfasst.

6. Verfahren zum Verbessern der Stabilität einer Weichgelatinekapsel, wobei das Verfahren die folgenden Schritte umfasst:
(a) Formulieren einer Arzneimittelzusammensetzung, umfassend:
i) von 55 Gew.-% bis 90 Gew.-% einen suspendierten Arzneimittelwirkstoff;
ii) von 0,001 Gew.-% bis 1,00 Gew.-% ein suspendiertes Stabilisierungsmittel, ausgewählt aus Dinatriumsalzen von Ethylendiamintetraessigsäure, Calciumsalzen von Ethylendiamintetraessigsäure, Tetranatriumethylendiamintetraessigsäure, und Mischungen davon; und
iii) von 9 Gew.-% bis 39 Gew.-% Lösungsmittel, ausgewählt aus Polyethylenglykolen; und
(b) Einkapseln der Zusammensetzung von (a) innerhalb der Weichgelatinekapsel.

7. Verfahren nach Anspruch 6, wobei der suspendierte Arzneimittelwirkstoff ausgewählt ist aus Antitussiva, Antihistaminen, nicht-sedierenden Antihistaminen, Dekongestiva, Expektoranzien, Mukolytika, Analgetika, Antipyretika, entzündungshemmenden Mitteln, Lokalanästhetika, und Mischungen davon.

8. Verfahren nach Anspruch 6 oder 7, wobei die Arzneimittelzusammensetzung ferner von 0,1 Gew.-% bis 5 Gew.-% Wasser umfasst.

## Revendications

1. Composition pharmaceutique comprenant :
(a) de 55 % à 90 % en poids d'un agent actif pharmaceutique en suspension ;
(b) de 0,001 % à 1,00 % en poids d'un agent stabilisant en suspension choisi parmi des sels disodiques d'acide éthylène diamine tétra-acétique, des sels de calcium d'acide éthylène diamine tétra-acétique, de l'acide éthylène diamine tétra-acétique tétrasodique, et des mélanges de ceux-ci ; et
(c) de 9 % à 39 % en poids d'un solvant choisi parmi des polyéthylène glycols ;
dans laquelle la composition est encapsulée au sein d'une capsule en gélatine molle.

2. Composition selon la revendication 1, dans laquelle la composition comprend de 58 % à 80 % en poids, de l'agent actif pharmaceutique en suspension choisi parmi des antitussifs, des antihistaminiques, des antihistaminiques non sédatifs, des décongestionnants, des expectorants, des mucolytiques, des analgésiques, des antipyrétiques, des agents anti-inflammatoires, des anesthésiques locaux, et des mélanges de ceux-ci.

3. Composition selon la revendication 1 ou la revendication 2, dans laquelle la composition comprend de 0,01 % à 1,00 % en poids de l'agent stabilisant en suspension.

4. Composition selon l'une quelconque des revendications précédentes, dans laquelle la composition comprend de 20 % à 39 % en poids de polyéthylène glycols.

5. Composition selon l'une quelconque des revendications précédentes, dans laquelle la composition comprend en outre de 0,1 % à 5 % en poids, d'eau.

6. Procédé d'amélioration de la stabilité d'une capsule en gélatine molle, dans lequel le procédé comprend les étapes consistant à :
(a) formuler une composition pharmaceutique comprenant :
i) de 55 % à 90 % en poids d'un agent actif pharmaceutique en suspension ;
ii) de 0,001 % à 1,00 % en poids d'un agent stabilisant en suspension choisi parmi des sels disodiques d'acide éthylène diamine tétra-acétique, des sels de calcium d'acide éthylène diamine tétra-acétique, de l'acide éthylène diamine tétra-acétique tétrasodique, et des mélanges de ceux-ci ; et
iii) de 9 % à 39 % en poids d'un solvant choisi parmi des polyéthylène glycols ; et
(b) encapsuler la composition de (a) au sein de la capsule en gélatine molle.

7. Procédé selon la revendication 6, dans lequel l'agent actif pharmaceutique en suspension est choisi parmi des antitussifs, des antihistaminiques, des antihistaminiques non sédatifs, des décongestionnants, des expectorants, des mucolytiques, des analgésiques, des antipyrétiques, des agents anti-inflammatoires, des anesthésiques locaux, et des mélanges de ceux-ci.

8. Procédé selon la revendication 6 ou 7, dans lequel la composition pharmaceutique comprend en outre de 0,1 % à 5 % en poids, d'eau.
